# EUROPÄISCHE PATENTANMELDUNG

(11) **EP 2 950 130 A1**
(43) Veröffentlichungstag der Anmeldung: **02.12.2015**
(21) Anmeldenummer: 15168020.4
(22) Anmeldetag: 18.05.2015
(51) Int. Cl.: G02B 21/00, G02B 21/36, A61B 19/00

(54) **MIKROSKOP-SYSTEM MIT TIEFENVORSCHAU**

(30) Priorität: 27.05.2014 DE 102014107443
(71) Anmelder: Carl Zeiss Meditec AG, 07745 Jena (DE)
(72) Erfinder: Saur, Stefan, 73430 Aalen (DE); Panitz, Gerald, 73479 Ellwangen (DE); Guckler, Roland, 89081 Ulm (DE)
(74) Vertreter: PATERIS Patentanwälte PartmbB

(57) **Zusammenfassung**

Es wird ein Mikroskop-System beschrieben, welches ein Mikroskop (2) zum Erzeugen eines mikroskopischen Bildes eines zu untersuchenden Beobachtungsbereiches (1), eine Anzeigeeinheit (60) zur Visualisierung des mikroskopischen Bildes, eine Registrierungseinheit (61) und eine Auswertungseinheit (62) umfasst. Die Registrierungseinheit (61) ist dazu ausgelegt die dreidimensionale Struktur eines Beobachtungsobjekts (12) von vorhandenen Daten auf die Position des Beobachtungsobjekts in dem Beobachtungsbereich (1) zu registrieren. Die Auswertungseinheit (62) ist dazu ausgelegt aus vorhandenen Daten eine Tiefenvorschaukarte (10) der dreidimensionalen Struktur des Beobachtungsobjekts zu berechnen und zur Anzeigeeinheit (60) zur Visualisierung der dreidimensionalen Struktur in Bezug auf die Position des Beobachtungsobjekts (12) in dem Beobachtungsbereich (1) zu übertragen.

## Beschreibung

### Gegenstand der Erfindung

Die vorliegende Erfindung betrifft ein Mikroskop-System, beispielsweise ein Operationsmikroskop-System, insbesondere für neurochirurgische Anwendungen. Die Erfindung betrifft weiterhin ein Mikroskopieverfahren, beispielsweise für Operationsmikroskope, insbesondere für neurochirurgische Anwendungen.

### Hintergrund der Erfindung

Eine Tumorresektion mittels Operationsmikroskop stellt eine generelle Herausforderung in der Chirurgie dar. Insbesondere bei der Resektion in funktionalen Gebieten muss die exakte dreidimensionale Morphologie des Tumors für den Chirurgen bekannt sein, um eine möglichst exakte Schnittgrenze zwischen funktionalem Gewebe und malignem Gewebe während einer Intervention wählen zu können.

Mit aktuell verfügbaren Navigationslösungen wird der Tumorrand, welcher basierend auf prä-operativen Daten berechnet wird, beispielsweise aus MRI-Untersuchungen, nur für die aktuelle Fokusebene bzw. Fokusschicht bzw. einzelne benachbarte Schichten durch das Okular im Operationsmikroskop angezeigt.

Für eine bessere Darstellung und Erkennung der dreidimensionalen Tumormorphologie muss der Chirurg eine entsprechende Morphologie entweder aus dem Gedächtnis abrufen oder er muss während der Intervention seinen gewohnten Blick durch das Okular des Operationsmikroskops aufgeben, um eine externe Visualisierungseinheit zu betrachten. Sowohl die vorab gelernte Morphologie als auch der kurzzeitige Blick an eine externe Visualisierungseinheit stellen Risiken für den Patienten dar, da der Operationsfluss unterbrochen wird.

Es ist daher Aufgabe der vorliegenden Erfindung ein vorteilhaftes Mikroskop-System und ein vorteilhaftes Mikroskopieverfahren zur Verfügung zu stellen.

### Beschreibung der Erfindung

Die oben genannte Aufgabe der vorliegenden Erfindung wird durch ein Mikroskop-System nach Patentanspruch 1 und durch ein Mikroskopieverfahren nach Patentanspruch 7 gelöst. Die abhängigen Ansprüche enthalten weitere, vorteilhafte Ausgestaltungen der Erfindung.

Das erfindungsgemäße Mikroskop-System umfasst ein Mikroskop, beispielsweise ein Operationsmikroskop, zum Erzeugen eines mikroskopischen Bildes eines zu untersuchenden Beobachtungsbereichs. Es umfasst weiterhin eine Anzeigeeinheit, beispielsweise eine Visualisierungseinheit oder ein Display, zur Visualisierung des mikroskopischen Bildes. Das Mikroskop-System umfasst zudem eine Registrierungseinheit und eine Auswertungseinheit. Das Mikroskop, die Anzeigeeinheit, die Registrierungseinheit und die Auswertungseinheit sind miteinander verbunden, insbesondere zur Datenübertragung.

Das erfindungsgemäße Mikroskop-System zeichnet sich dadurch aus, dass die Registrierungseinheit dazu ausgelegt ist, die dreidimensionale Struktur eines Beobachtungsobjekts von vorhandenen Daten, beispielsweise zu einem früheren Zeitpunkt gewonnenen Daten, auf die Position des Beobachtungsobjekts in dem Beobachtungsbereich zu übertragen. Bei dem Beobachtungsobjekt kann es sich beispielsweise um Gewebe, insbesondere Gewebe eines Tumors, handeln. Die Registrierungseinheit kann also dazu ausgelegt sein, die Morphologie des Gewebes, beispielsweise die Tumormorphologie, von präoperativen Daten auf den aktuellen Beobachtungsbereich bzw. die aktuelle Operationsszene zu registrieren.

Das erfindungsgemäße Mikroskop-System zeichnet sich weiterhin dadurch aus, dass die Auswertungseinheit dazu ausgelegt ist, aus vorhandenen Daten, beispielsweise zu einem früheren Zeitpunkt gewonnenen Daten, eine Tiefenvorschaukarte der dreidimensionalen Struktur des Beobachtungsobjekts zu berechnen und zur Anzeigeeinheit zur Visualisierung der dreidimensionalen Struktur in Bezug auf die Position des Beobachtungsobjekts in dem Beobachtungsbereich zu übertragen. Beispielsweise kann die Auswertungseinheit dazu ausgelegt sein, aus vorhandenen Daten die Morphologie eines Gewebes, insbesondere Tumors, zu berechnen und zur Anzeigeeinheit zur Visualisierung der dreidimensionalen Struktur in Bezug auf die Position des Beobachtungsobjekts in dem Beobachtungsbereich zu übertragen. Bei der Auswertungseinheit kann es sich beispielsweise um einen PC handeln.

Das oben in Zusammenhang mit Tumorresektion beschriebene Problem der unzureichenden Visualisierungsmöglichkeit eines Tumors während eines operativen Eingriffs kann mit Hilfe des erfindungsgemäßen Mikroskop-Systems durch eine Tumortiefenvorschaukarte gelöst werden, welche die gesamte Morphologie des Tumors beispielsweise als Iso-Tiefenlinien (aka Isobathen) visualisiert, die direkt in das Okular des Operationsmikroskops eingeblendet wird. Zur besseren Unterscheidung können einzelne charakteristische Tiefenbereiche optional noch farblich codiert werden. Der Chirurg muss somit seinen gewohnten Blick durch das Okular des Operationsmikroskops nicht aufgeben. Er muss sich auch nicht auf eine vorab gelernte Morphologie verlassen. Dies verbessert den Operationsfluss und reduziert die oben genannten Risiken für den Patienten.

Grundsätzlich können im Rahmen des erfindungsgemäßen Mikroskop-Systems Techniken, wie beispielsweise die Fusionierung von Bilddaten, aus dem Bereich der Augmented Reality zum Einsatz kommen, um eine nahtlose und realitätsgetreue Einblendung in die Operationsszene zu ermöglichen.

Vorteilhafterweise umfasst das Mikroskop-System ein Messsystem, welches ausgelegt ist zur Erfassung der Topographie des Beobachtungsbereichs, insbesondere der aktuellen Operationsszene. Das Messsystem kann einen stereoskopischen Sensor und/oder einen Laserscanner und/oder einen Sensor zur Laufzeitmessung, beispielsweise eine Time-off-Flight-Kamera (TOF-Kamera), und/oder eine Vorrichtung zur strukturierten Beleuchtung umfassen. Der stereoskopische Sensor kann zum Beispiel zwei in dem chirurgischen Mikroskop integrierte Kameras umfassen.

Optional umfasst das Mikroskop-System ein Visualisierungssystem, beispielsweise in Form einer Videokamera, welches dazu ausgelegt ist, Bilder, beispielsweise aktuelle Bilder, des Beobachtungsbereichs, insbesondere der Operationsszene zu erfassen, um diese mit der Tiefenvorschaukarte zu kombinieren. Grundsätzlich kann die Visualisierung der Tiefenvorschaukarte bzw. Tiefenkarte als opake Überlagerung erfolgen.

Eine Alternative Form der Visualisierung ist die linienhafte Anzeige der Iso-Tiefenlinien, wobei die Linien solide, gestrichelt, punktiert oder in einer beliebigen Kombination davon gezeigt werden können. Denkbar ist auch jegliche Form einer Überblendung von Iso-Tiefenlinien bzw. opaker Darstellung mit der aktuellen Operationsszene bzw. dem Beobachtungsbereich um ein realistisches "Look-and-Feel" zu ermöglichen. Vorteilhafterweise können für sichtbare bzw. für nicht sichtbare Bereiche des malignen Gewebes unterschiedliche Visualisierungsparameter gewählt werden.

In einer weiteren Ausprägung können die Tiefeninformationen auch lokal begrenzt angezeigt werden, um das Sichtfeld der untersuchenden Person, beispielsweise des Operateurs, nicht zu beschränken.

Vorzugsweise erfolgt die Visualisierung primär im Okular des Operationsmikroskops. Alternativ oder zusätzlich kann die Visualisierung auch auf einer externen Anzeigeeinheit, beispielsweise einem Monitor, einer Datenbrille oder ähnlichem, erfolgen.

Die Registrierungseinheit kann ein Navigationsgerät umfassen. Sie kann grundsätzlich zur rigiden oder zur nicht rigiden Übertragung bzw. Registrierung ausgelegt sein. Die Registrierungseinheit kann zum Beispiel in der Form eines Navigationsgerätes ausgestaltet sein.

Wie bereits erwähnt kann die Anzeigeeinheit ein okulares oder externes Display umfassen. Weiterhin kann die Anzeigeeinheit zur Visualisierung der Tiefenvorschaukarte bzw. Tiefenkarte als opake Überlagerung und/oder zur Visualisierung der Tiefenvorschaukarte bzw. Tiefenkarte in Form von Iso-Tiefenlinien ausgelegt sein. Bei der extern ausgestalteten Anzeigeeinheit kann es sich zum Beispiel um einen Monitor oder eine Datenbrille handeln.

Insgesamt hat das erfindungsgemäße Mikroskop-System den Vorteil, dass es eine verbesserte Darstellung der Gesamtmorphologie eines Beobachtungsobjektes innerhalb eines Beobachtungsbereichs, beispielsweise eine verbesserte Darstellung der Gesamtmorphologie von malignem Gewebe innerhalb einer Operationsszene, ermöglicht.

Im Rahmen des erfindungsgemäßen Mikroskopie-Verfahrens wird mit einem Mikroskop ein mikroskopisches Bild eines zu untersuchenden Beobachtungsbereiches erzeugt und mit einer Anzeigeeinheit visualisiert. Die dreidimensionale Struktur eines Beobachtungsobjektes, beispielsweise eines Gewebebereiches, wird von vorhandenen Daten, insbesondere zu einem früheren Zeitpunkt gewonnenen prä-operativen Daten, auf die Position des Beobachtungsobjekts in dem Beobachtungsbereich, insbesondere auf den aktuellen Beobachtungsbereich bzw. die aktuelle Operationsszene, übertragen bzw. registriert. Weiterhin wird aus vorhandenen, beispielsweise zu einem früheren Zeitpunkt gewonnenen prä-operativen Daten, eine Tiefenvorschaukarte einer dreidimensionalen Struktur des Beobachtungsobjektes, beispielsweise des Gewebebereiches berechnet. Die berechnete dreidimensionale Struktur wird in Bezug auf die Position des Beobachtungsobjektes in den Beobachtungsbereich mit Hilfe der Anzeigeeinheit visualisiert.

Das erfindungsgemäße Verfahren kann beispielsweise mit Hilfe des zuvor beschriebenen erfindungsgemäßen Mikroskop-Systems durchgeführt werden. Es hat grundsätzlich die Vorteile wie das zuvor beschriebene erfindungsgemäße Mikroskop-System.

Vorteilhafterweise können eine im Rahmen des erfindungsgemäßen Mikroskop-Systems beschriebene Registrierungseinheit und/oder eine dort beschriebene Auswertungseinheit Verwendung finden. Bei dem Beobachtungsbereich handelt es sich vorzugsweise um eine chirurgische, beispielsweise neurochirurgische, Operationsszene.

Vorteilhafterweise kann die Topographie des Beobachtungsbereiches, beispielsweise die aktuelle Operationsszene, erfasst werden. Hierzu kann ein entsprechendes Messsystem verwendet werden, welches zur Erfassung der Topographie des Beobachtungsbereiches ausgelegt ist. Die Erfassung kann prinzipiell stereoskopisch, beispielsweise mit Hilfe eines stereoskopischen Sensors, insbesondere mit Hilfe von zwei im chirurgischen Mikroskop integrierten Videokameras, und/oder mit Hilfe eines Laserscanners und/oder mit Hilfe eines Verfahrens zur Laufzeitmessung, beispielsweise mit Hilfe eines Sensors zur Laufzeitmessung wie vorzugsweise einer TOF-Kamera, erfolgen. Alternativ dazu oder zusätzlich kann die Topographieerfassung durch eine strukturierte Beleuchtung, beispielsweise mit Hilfe einer Vorrichtung zur strukturierten Beleuchtung, erfolgen.

Durch die Miteinbeziehung aktueller Topographieinformationen können die Tiefeninformationen sowie deren Visualisierung den aktuellen Gegebenheiten angepasst werden. In einer anderen Erweiterung werden die intraoperativen Bilddaten dazu verwendet, eine mögliche geographische Abweichung von prä- und intraoperativen Daten für die Visualisierung der Tiefeninformationen zu kompensieren. Dazu können verschiedene Bildverarbeitungsalgorithmen und/oder verschiedene Beleuchtungsmodi und/oder Marker, z.B. Kontrastmittel, zum Einsatz kommen.

Grundsätzlich können die Tiefeninformationen mit Hilfe von externen Navigationslösungen erfasst werden. Konkret können die Tiefeninformationen mittels MRI (Magnetresonanztomographie), CT (Computertomographie) oder Ähnlichem erfasst werden. Das Navigationssystem kann die Daten segmentieren, also beispielsweise Knochen oder Tumorgewebe zuordnen, und kann sie entweder als Rohdaten oder bereits rechnerisch auf die optische Achse des Operationsmikroskops korrigiert liefern.

In einer Ausprägung davon stellt das Navigationssystem die gesamten Tiefeninformationen über eine Schnittstelle bereit. In einer anderen Ausprägung sendet das Operationsmikroskop basierend auf der aktuellen Fokusebene "virtuelle Tiefen" (Abstand des verstellbaren Bildfokus relativ zum Operationsmikroskop) in einem definierten Wertebereich an eine externe Navigationslösung, um die jeweilige Kontur des malignen Gewebes im prä-operativen Zustand für die gesendete Tiefe zu erlangen. Diese Konturen werden dann im Operationsmikroskop zu der Tiefenkarte zusammengefasst.

Optional werden die mikroskopisch erzeugten Bilder des zu untersuchenden Beobachtungsbereiches, beispielsweise der Operationsszene, erfasst. Diese werden anschließend mit der Tiefenvorschaukarte kombiniert. Hierzu kann insbesondere ein entsprechend ausgelegtes Visualisierungssystem, beispielsweise eine Videokamera, verwendet werden.

Im Rahmen des Mikroskopie-Verfahrens kann die Tiefenvorschaukarte bzw. Tiefenkarte als opake Überlagerung und/oder in Form von Iso-Tiefenlinien visualisiert werden, beispielsweise mit Hilfe einer Anzeigeeinheit. Die verwendete Anzeigeeinheit kann ein okulares oder externes Display umfassen. Sie kann insbesondere zur Visualisierung der Tiefenkarte als opake Überlagerung und/oder zur Visualisierung in Form von Iso-Tiefenlinien ausgelegt sein. Als externe Anzeigeeinheit kann insbesondere ein Monitor oder eine Datenbrille verwendet werden.

Die Übertragung bzw. Registrierung der dreidimensionalen Struktur des Beobachtungsobjekts auf der Basis von vorhandenen Daten auf die Position des Beobachtungsobjekts in dem Beobachtungsbereich kann prinzipiell rigide oder nicht-rigide erfolgen. Hierzu kann eine Registrierungseinheit verwendet werden, die zur rigiden oder zur nicht-rigiden Übertragung bzw. Registrierung ausgelegt ist.

### Beschreibung von Ausführungsbeispielen

Weitere Merkmale, Eigenschaften und Vorteile der vorliegenden Erfindung werden im Folgenden anhand von Ausführungsbeispielen unter Bezugnahme auf die beigefügten Figuren näher beschrieben. Alle bisher und im Folgenden beschriebenen Merkmale sind dabei sowohl einzeln als auch in einer beliebigen Kombination miteinander vorteilhaft. Die im Folgenden beschriebenen Ausführungsbeispiele stellen lediglich Beispiele dar, welche den Gegenstand der Erfindung jedoch nicht beschränken.
- Fig. 1: zeigt schematisch den Aufbau eines Operationsmikroskops.
- Fig. 2: zeigt schematisch Beispiel für ein Vario-Objektiv.
- Fig. 3: zeigt schematisch eine chirurgische Operationsszene mit Tiefenvorschaukarte.
- Fig. 4: zeigt schematisch ein erfindungsgemäßes Mikroskop-System.

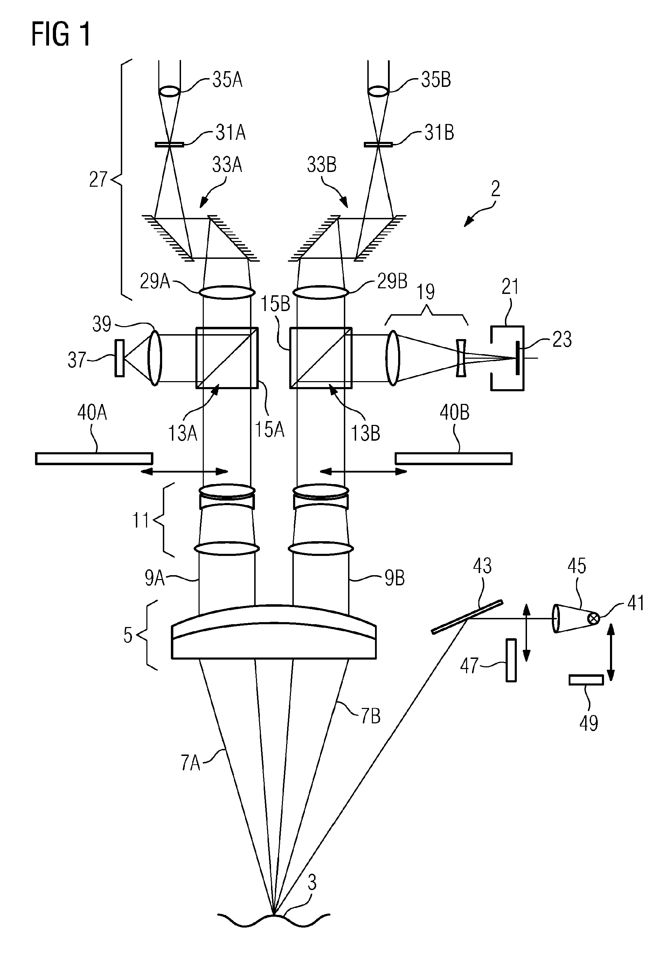

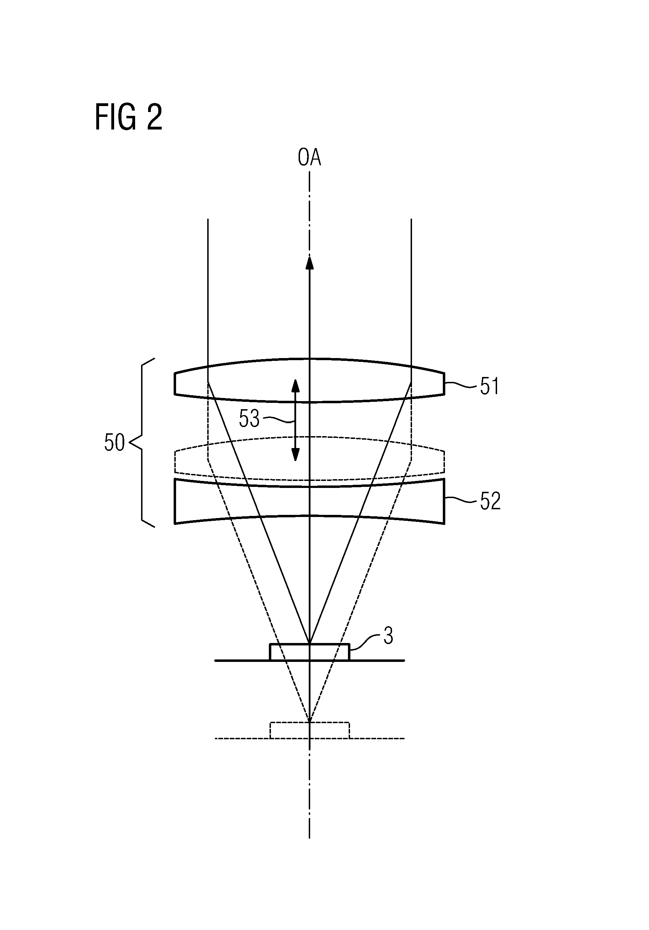

Nachfolgend wird mit Bezug auf die Figuren 1 und 2 der grundsätzliche Aufbau des Operationsmikroskops 2 erläutert.

Das in Figur 1 gezeigte Operationsmikroskop 2 umfasst als wesentliche Bestandteile ein einem Objektfeld 3 zuzuwendendes Objektiv 5, das insbesondere als achromatisches oder apochromatisches Objektiv ausgebildet sein kann. Im vorliegenden Ausführungsbeispiel besteht das Objektiv 5 aus zwei miteinander verkitteten Teillinsen, die ein achromatisches Objektiv bilden. Das Objektfeld 3 wird in der Brennebene des Objektivs 5 angeordnet, so dass es vom Objektiv 5 nach Unendlich abgebildet wird. Mit anderen Worten, ein vom Objektfeld 3 ausgehendes divergentes Strahlenbündel 7 wird bei seinem Durchgang durch das Objektiv 5 in ein paralleles Strahlenbündel 9 umgewandelt.

Beobachterseitig des Objektivs 5 ist ein Vergrößerungswechsler 11 angeordnet, der entweder wie im dargestellten Ausführungsbeispiel als Zoom-System zur stufenlosen Änderung des Vergrößerungsfaktors oder als so genannter Galilei-Wechsler zur stufenweisen Änderung des Vergrößerungsfaktors ausgebildet sein kann. In einem Zoom-System, das bspw. aus einer Linsenkombination mit drei Linsen aufgebaut ist, können die beiden objektseitigen Linsen verschoben werden, um den Vergrößerungsfaktor zu variieren. Tatsächlich kann das Zoom-System aber auch mehr als drei Linsen, bspw. vier oder mehr Linsen aufweisen, wobei die äußeren Linsen dann auch fest angeordnet sein können. In einem Galilei-Wechsler existieren dagegen mehrere feste Linsenkombinationen, die unterschiedliche Vergrößerungsfaktoren repräsentieren und im Wechsel in den Strahlengang eingebracht werden können. Sowohl ein Zoom-System, als auch ein Galilei-Wechsler wandeln ein objektseitiges paralleles Strahlenbündel in ein beobachterseitiges paralleles Strahlenbündel mit einem anderen Bündeldurchmesser um. Der Vergrößerungswechsler 11 ist im vorliegenden Ausführungsbeispiel bereits Teil des binokularen Strahlengangs des Operationsmikroskops 1, d.h. er weist eine eigene Linsenkombination für jeden stereoskopischen Teilstrahlengang 9A, 9B des Operationsmikroskops 1 auf. Das Einstellen eines Vergrößerungsfaktors mittels des Vergrößerungswechslers 11 erfolgt im vorliegenden Ausführungsbeispiel über ein motorisch angetriebenes Stellglied, das zusammen mit dem Vergrößerungswechsler 11 Teil einer Vergrößerungswechseleinheit zum Einstellen des Vergrößerungsfaktors ist.

An den Vergrößerungswechsler 11 schließt sich beobachterseitig eine Schnittstellenanordnung 13A, 13B an, über die externe Geräte an das Operationsmikroskop 1 angeschlossen werden können und die im vorliegenden Ausführungsbeispiel Strahlteilerprismen 15A, 15B umfasst. Grundsätzlich können aber auch andere Arten von Strahlteilern Verwendung finden, bspw. teildurchlässige Spiegel. Die Schnittstellen 13A, 13B dienen im vorliegenden Ausführungsbeispiel zum Auskoppeln eines Strahlenbündels aus dem Strahlengang des Operationsmikroskops 2 (Strahlteilerprisma 15B) bzw. zum Einkoppeln eines Strahlenbündels in den Strahlengang des Operationsmikroskops 2 (Strahlteilerprisma 15A).

Das Strahlteilerprisma 15A in dem Teilstrahlengang 9A dient im vorliegenden Ausführungsbeispiel dazu, mit Hilfe eines Displays 37, bspw. einer Digital Mirror Device (DMD) oder eines LCD-Displays, und einer zugehörigen Optik 39 über das Strahlteilerprisma 15A Informationen oder Daten für einen Betrachter in den Teilstrahlengang 9A des Operationsmikroskops 1 einzuspiegeln. Im anderen Teilstrahlengang 9B ist an der Schnittstelle 13B ein Kameraadapter 19 mit einer daran befestigten Kamera 21 angeordnet, die mit einem elektronischen Bildsensor 23, bspw. mit einem CCD-Sensor oder einem CMOS-Sensor, ausgestattet ist. Mittels der Kamera 21 kann ein elektronisches und insbesondere ein digitales Bild des Gewebebereichs 3 aufgenommen werden. Als Bildsensor kann insbesondere auch ein Hyperspektralsensor Verwendung finden, in dem nicht nur drei Spektralkanäle (bspw. rot, grün und blau) vorhanden sind, sondern eine Vielzahl von Spektralkanälen.

An die Schnittstelle 13 schließt sich beobachterseitig ein Binokulartubus 27 an. Dieser weist zwei Tubusobjektive 29A, 29B auf, welche das jeweilige parallele Strahlenbündel 9A, 9B auf eine Zwischenbildebene 31 fokussieren, also das Beobachtungsobjekt 3 auf die jeweilige Zwischenbildebene 31A, 31 B abbilden. Die in den Zwischenbildebenen 31A, 31 B befindlichen Zwischenbilder werden schließlich von Okularlinsen 35A, 35B wiederum nach Unendlich abgebildet, so dass ein Betrachter das Zwischenbild mit entspanntem Auge betrachten kann. Außerdem erfolgt im Binokulartubus mittels eines Spiegelsystems oder mittels Prismen 33A, 33B eine Vergrößerung des Abstandes zwischen den beiden Teilstrahlenbündeln 9A, 9B, um diesen an den Augenabstand des Betrachters anzupassen. Mit dem Spiegelsystem oder den Prismen 33A, 33B erfolgt zudem eine Bildaufrichtung.

Das Operationsmikroskop 2 ist außerdem mit einer Beleuchtungsvorrichtung ausgestattet, mit der der das Objektfeld 3 mit breitbandigem Beleuchtungslicht beleuchtet werden kann. Hierzu weist die Beleuchtungsvorrichtung im vorliegenden Ausführungsbeispiel eine Weißlichtquelle 41, etwa eine Halogenglühlampe oder eine Gasentladungslampe, auf. Das von der Weißlichtquelle 41 ausgehende Licht wird über einen Umlenkspiegel 43 oder ein Umlenkprisma in Richtung auf das Objektfeld 3 gelenkt, um dieses auszuleuchten. In der Beleuchtungsvorrichtung ist weiterhin eine Beleuchtungsoptik 45 vorhanden, die für eine gleichmäßige Ausleuchtung des gesamten beobachteten Objektfeldes 3 sorgt.

Es sei darauf hingewiesen, dass der in Figur 1 dargestellte Beleuchtungsstrahlengang stark schematisiert ist und nicht notwendigerweise den tatsächlichen Verlauf des Beleuchtungsstrahlengangs wiedergibt. Grundsätzlich kann der Beleuchtungsstrahlengang als sogenannte Schrägbeleuchtung ausgeführt sein, die der schematischen Darstellung in Figur 1 am nächsten kommt. In einer solchen Schrägbeleuchtung verläuft der Strahlengang in einem relativ großen Winkel (6° oder mehr) zur optischen Achse des Objektivs 5 und kann, wie in Figur 1 dargestellt, vollständig außerhalb des Objektivs verlaufen. Alternativ besteht jedoch auch die Möglichkeit, den Beleuchtungsstrahlengang der Schrägbeleuchtung durch einen Randbereich des Objektivs 5 hindurch verlaufen zu lassen. Eine weitere Möglichkeit zur Anordnung des Beleuchtungsstrahlengangs ist die sogenannte 0°-Beleuchtung, bei der der Beleuchtungsstrahlengang durch das Objektiv 5 hindurch verläuft und zwischen den beiden Teilstrahlengängen 9A, 9B, entlang der optischen Achse des Objektivs 5 in Richtung auf das Objektfeld 3 in das Objektiv eingekoppelt wird. Schließlich besteht auch die Möglichkeit, den Beleuchtungsstrahlengang als sogenannte koaxiale Beleuchtung auszuführen, in der ein erster und ein zweiter Beleuchtungsteilstrahlengang vorhanden sind. Die Teilstrahlengänge werden über einen oder mehrere Strahlteiler parallel zu den optischen Achsen der Beobachtungsteilstrahlengänge 9A, 9B in das Operationsmikroskop eingekoppelt, so dass die Beleuchtung koaxial zu den beiden Beobachtungsteilstrahlengängen verläuft.

In dem in Figur 1 dargestellten Operationsmikroskop kann auf die Beleuchtung Einfluss genommen werden. Bspw. kann ein Filter 47 in den Beleuchtungsstrahlengang eingebracht werden, der von dem breiten Spektrum der Weißlichtquelle 41 nur einen schmalen Spektralbereich passieren lässt, bspw. einen Spektralbereich, mit dem Fluoreszenz eines im Objektfeld 3 befindlichen Fluoreszenzfarbstoffes angeregt werden kann. Zur Beobachtung der Fluoreszenz können in die Beobachtungs-Teilstrahlengänge Filter 37A, 37B eingebracht werden, die den zur Fluoreszenzanregung verwendeten Spektralbereich herausfiltern um die Fluoreszenz beobachten zu können.

Die Beleuchtungsvorrichtung kann zudem mit einer Einheit zum Wechsel der Beleuchtungslichtquelle ausgestattet sein. Diese ist in Figur 1 durch ein System zum Austausch der Weißlichtquelle 41 durch eine Laser 49 angedeutet. Mit einem Laser als Lichtquelle, insbesondere mit einem Infrarotlaser, wird in Verbindung mit einem geeigneten Bildsensor 23 bspw. Laser-Doppler-Imaging oder Laser-Speckle-Imaging ermöglicht. Die Einheit zum Wechsel der Beleuchtungslichtquelle ist im vorliegenden Ausführungsbeispiel motorisch angetrieben und kann mittels geeigneter Steuerdaten von der Pathologieeinheit 70 aus gesteuert werden

In der in Figur 1 gezeigten Ausführungsvariante des Operationsmikroskops 2 besteht das Objektiv 5 lediglich aus einer Achromatlinse. Es kann jedoch auch ein Objektivlinsensystem aus mehreren Linsen Verwendung finden, insbesondere ein so genanntes Vario-Objektiv, mit dem sich der Arbeitsabstand des Operationsmikroskops 2, d.h. der Abstand der objektseitigen Brennebene vom Scheitel der ersten objektseitigen Linsenfläche des Objektivs 5, auch Objektschnittweite genannt, variieren lässt. Auch vom Vario-Objektiv 50 wird das in der Brennebene angeordnete Objektfeld 3 nach Unendlich abgebildet, so dass beobachterseitig ein paralleles Strahlenbündel vorliegt.

Ein Beispiel für ein Vario-Objektiv ist schematisch in Figur 2 dargestellt. Das Vario-Objektiv 50 umfasst ein Positivglied 51, also ein optisches Element mit positiver Brechkraft, das in Figur 2 schematisch als Konvexlinse dargestellt ist. Darüber hinaus umfasst das Vario-Objektiv 50 ein Negativglied 52, also ein optisches Element mit negativer Brechkraft, das in Figur 2 schematisch als Konkavlinse dargestellt ist. Das Negativglied 52 befindet sich zwischen dem Positivglied 51 und dem Objektfeld 3. Im dargestellten Vario-Objektiv 50 ist das Negativglied 52 fix angeordnet, wohingegen das Positivglied 51 wie durch den Doppelpfeil 53 angedeutet entlang der optischen Achse OA verschiebbar angeordnet ist. Wenn das Positivglied 51 in die in Figur 2 gestrichelt dargestellte Position verschoben wird, verlängert sich die Schnittweite, so dass sich der Arbeitsabstand des Operationsmikroskops 2 vom Objektfeld 3 ändert.

Obwohl in Figur 2 das Positivglied 51 verschiebbar ausgestaltet ist, besteht grundsätzlich auch die Möglichkeit, das Negativglied 52 statt des Positivglieds 51 entlang der optischen Achse OA bewegbar anzuordnen. Das Negativglied 52 bildet jedoch häufig die Abschlusslinse des Vario-Objektivs 50. Ein feststehendes Negativglied 52 bietet daher den Vorteil, dass das Innere des Operationsmikroskops 2 leichter gegen äußere Einflüsse abgedichtet werden kann. Weiterhin sei angemerkt, dass, obwohl das Positivglied 51 und das Negativglied 52 in Figur 2 lediglich als Einzellinsen dargestellt sind, jedes dieser Glieder statt in Form einer Einzellinse auch in Form einer Linsengruppe oder eines Kittglieds realisiert sein kann, bspw. um das Vario-Objektiv achromatisch oder apochromatisch auszubilden.

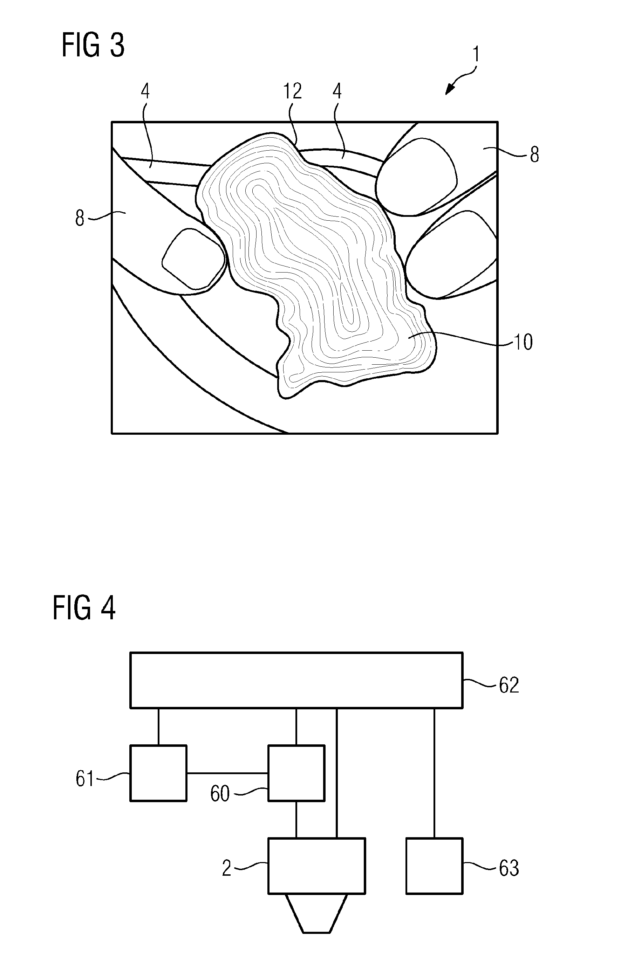

Die Figur 3 zeigt schematisch eine chirurgische, beispielsweise eine neurochirurgische Operationsszene 1. Der durch das Operationsmikroskop mikroskopisch dargestellte Beobachtungsbereich bzw. die Operationsszene umfasst zu entfernendes Gewebe 12, beispielsweise im Rahmen einer Tumorresektion zu entfernendes Tumorgewebe. Die Finger des Chirurgen sind mit der Bezugsziffer 8 gekennzeichnet, das verwendete Operationsbesteck ist mit der Bezugsziffer 4 gekennzeichnet. Während der Operation wird gemäß dem erfindungsgemäßen Verfahren eine Tumortiefenvorschaukarte 10 in die Operationsszene eingeblendet. Dies erfolgt vorliegend mit Hilfe von Iso-Tiefenlinien und durch farbliche Codierung.

Grundsätzlich können dabei Techniken aus dem Bereich der Augmented Reality zur Fusionierung von Bilddaten verwendet werden. Dies ermöglicht eine nahtlose und realitätsgetreue Einblendung in die Operationsszene.

Mit Hilfe der Tumortiefenvorschaukarte 10 wird die gesamte Morphologie des Tumors 12 als Iso-Tiefenlinien, wie in der Figur 3 gezeigt, visualisiert und direkt in das Okular des Operationsmikroskops eingeblendet. Zur besseren Unterscheidung können einzelne charakteristische Tiefenbereiche optional noch farblich codiert werden. Alternativ zu einer direkten Einblendung in das Okular des Operationsmikroskops oder zusätzlich hierzu ist eine Visualisierung mit Hilfe einer externen Anzeigeeinheit, beispielsweise einem Monitor, einer Datenbrille oder ähnlichem, möglich.

Die Tiefeninformationen werden zum Beispiel unter Zuhilfenahme von externen Navigationslösungen erfasst. Dabei kann das Navigationssystem die gesamten Tiefeninformationen über eine Schnittstelle bereitstellen. In einer anderen Ausgestaltung kann das Operationsmikroskop 2 zusätzlich oder alternativ dazu basierend auf der aktuellen Fokusebene "virtuelle Tiefen" in einem definierten Wertebereich an eine externe Navigationslösung senden, um die jeweilige Kontur des malignen Gewebes 12 im präoperativen Zustand für die gesendete Tiefe zu erlangen.

In einer erweiterten Variante der Topographieinformationen der Operationsszene durch einen geeigneten Sensor, beispielsweise einem stereoskopischen Sensor, einem Laserscanner, einem Time-of-Flight-Sensor oder mit Hilfe von strukturierter Beleuchtung erfasst. Der stereoskopische Sensor kann zum Beispiel zwei in dem chirurgischen Mikroskop integrierte Videokameras umfassen. Durch die Miteinbeziehung aktueller Topographieinformationen können die Tiefeninformationen sowie deren Visualisierung den aktuellen Gegebenheiten angepasst werden. Insbesondere können auftretende Deformationen berücksichtigt werden.

Als weitere Variante werden die intraoperativen Bilddaten dazu verwendet, eine mögliche geographische Abweichung von prä- und intraoperativen Daten für die Visualisierung der Tiefeninformationen zu kompensieren. Dazu können verschiedene Bildverarbeitungsalgorithmen, Beleuchtungsmodi und Marker, zum Beispiel Kontrastmittel, zum Einsatz kommen.

Grundsätzlich kann die Visualisierung der Tiefenvorschaukarte bzw. Tiefenkarte als opake Überlagerung, wie zum Beispiel in der Figur 3 gezeigt, erfolgen. Eine alternative Form der Visualisierung ist die linienhafte Anzeige der Iso-Tiefenlinien, wobei die Linien solide, gestrichelt, punktiert oder in einer beliebigen Kombination davon angezeigt werden. Möglich ist auch jegliche Form einer Überblendung von Iso-Tiefenlinien bzw. opaker Darstellung mit der aktuellen Beobachtungsszene bzw. Operationsszene um ein realistisches "Look-and-Feel" zu ermöglichen. Für sichtbare bzw. nicht sichtbare Bereiche des malignen Gewebes können unterschiedliche Visualisierungsparameter gewählt werden. In einer weiteren Ausprägung können die Tiefeninformationen auch lokal begrenzt angezeigt werden, um das Sichtfeld des Operateurs 8 nicht zu beschränken.

Bevorzugt erfolgt die Visualisierung primär im Okular des Operationsmikroskops, zum Beispiel als perspektivisch korrekte Überlagerung oder als Bild im Bild (PiP - Picture in Picture) am Rande des Sichtfeldes, um das Verdecken von relevanten Bildinformationen zu minimieren. Die Visualisierung kann optional aber auch auf einer externen Anzeigeeinheit erfolgen.

Die Figur 4 zeigt schematisch ein erfindungsgemäßes Mikroskop-System. Es umfasst ein Operationsmikroskop 2, eine Anzeigeeinheit 60, eine Registrierungseinheit 61 und eine Auswertungseinheit 62, die miteinander zur Datenübertragung verbunden sind. Die Anzeigeeinheit 60 kann als okulares oder externes Display ausgestaltet sein. Die Registrierungseinheit 61 kann beispielsweise in Form eines Navigationsgeräts ausgestaltet sein. Sie kann insbesondere ausgelegt sein zur Registrierung der Tumormorphologie von prä-operativen Daten auf die aktuelle Operationsszene. Dies kann in rigider oder nicht rigider Form erfolgen. Die Auswertungseinheit 62 ist dazu ausgelegt verschiedene Quellen der Tiefeninformationen zu kombinieren und daraus eine Tiefenvorschaukarte 10 zu berechnen. Hierzu kann die Auswertungseinheit 62 Algorithmen umfassen, welche Daten aus verschiedenen Quellen in Bezug auf die Tiefeninformationen kombinieren und daraus eine Tiefenvorschaukarte 10 berechnen. Die Auswertungseinheit 62 ist darüber hinaus dazu ausgelegt, die berechnete Tiefenvorschaukarte 10 zu der Anzeigeeinheit 60 zu senden.

Optional kann das Operationssystem ein Messsystem 63 umfassen, welches dazu ausgelegt ist, die Topographie der aktuellen Operationsszene zu erfassen. Ebenfalls optional kann das Mikroskop-System ein System, beispielsweise in Form einer Videokamera, umfassen, welches dazu ausgelegt ist, aktuelle Bilder der Operationsszene zu erfassen und diese mit der Tiefenvorschaukarte zu kombinieren.

### Bezugszeichenliste

- 1: Operationsszene
- 2: Operationsmikroskop
- 3: Operationsfeld
- 4: Operationsbesteck
- 5: Objektiv
- 7: divergentes Strahlenbündel
- 8: Finger des Chirurgen
- 9: Strahlenbündel
- 9A, 9B: stereoskopischer Teilstrahlengang
- 10: Tumortiefenvorschaukarte
- 12: Gewebe
- 11: Vergrößerungswechsler
- 13A, 13B: Schnittstellenanordnung
- 15A, 15B: Strahlteilerprisma
- 19: Kameraadapter
- 21: Kamera
- 23: Bildsensor
- 27: Binokulartubus
- 29A, 29B: Tubusobjektiv
- 31 A,: 31B Zwischenbildebene
- 33A, 33B: Prisma
- 35A, 35B: Okularlinse
- 37: Display
- 39: Optik
- 40A, 40B: Spektralfilter
- 41: Weißlichtquelle
- 43: Umlenkspiegel
- 45: Beleuchtungsoptik
- 47: Spektralfilter
- 49: Laser
- 50: Vario-Objektiv
- 51: Positivglied
- 52: Negativglied
- 53: Verschiebeweg
- 60: Anzeigeeinheit
- 61: Registrierungseinheit
- 62: Auswertungseinheit
- 63: Messsystem

## Patentansprüche

1. Mikroskop-System, welches ein Mikroskop (2) zum Erzeugen eines mikroskopischen Bildes eines zu untersuchenden Beobachtungsbereiches (1), eine Anzeigeeinheit (60) zur Visualisierung des mikroskopischen Bildes, eine Registrierungseinheit (61) und eine Auswertungseinheit (62) umfasst, wobei das Mikroskop (2), die Anzeigeeinheit (60), die Registrierungseinheit (61) und die Auswertungseinheit (62) miteinander verbunden sind,
**dadurch gekennzeichnet, dass**
- die Registrierungseinheit (61) dazu ausgelegt ist die dreidimensionale Struktur eines Beobachtungsobjekts (12) von vorhandenen Daten auf die Position des Beobachtungsobjekts in dem Beobachtungsbereich (1) zu registrieren,
- die Auswertungseinheit (62) dazu ausgelegt ist aus vorhandenen Daten eine Tiefenvorschaukarte (10) der dreidimensionalen Struktur des Beobachtungsobjekts zu berechnen und zur Anzeigeeinheit (60) zur Visualisierung der dreidimensionalen Struktur in Bezug auf die Position des Beobachtungsobjekts (12) in dem Beobachtungsbereich (1) zu übertragen.

2. Mikroskop-System nach Anspruch 1,
**dadurch gekennzeichnet, dass**
es ein Messsystem (63) umfasst, welches ausgelegt ist zur Erfassung der Topographie des Beobachtungsbereiches (1).

3. Mikroskop-System nach Anspruch 2,
**dadurch gekennzeichnet, dass**
das Messsystem (63) einen stereoskopischen Sensor und/oder einen Laserscanner und/oder einen Sensor zur Laufzeitmessung und/oder eine Vorrichtung zur strukturierten Beleuchtung umfasst.

4. Mikroskop-System nach einem der Ansprüche 1 bis 3,
**dadurch gekennzeichnet, dass**
es ein Visualisierungssystem umfasst, welches dazu ausgelegt ist Bilder des Beobachtungsbereiches (1) zu erfassen um diese mit der Tiefenvorschaukarte (10) zu kombinieren.

5. Mikroskop-System nach einem der Ansprüche 1 bis 4,
**dadurch gekennzeichnet, dass**
die Registrierungseinheit (61) ein Navigationsgerät umfasst und/oder zur rigiden oder nicht rigiden Registrierung ausgelegt ist.

6. Mikroskop-System nach einem der Ansprüche 1 bis 5,
**dadurch gekennzeichnet, dass**
die Anzeigeeinheit (60) ein okulares oder externes Display umfasst und/oder ausgelegt ist zur Visualisierung der Tiefenvorschaukarte (10) als opake Überlagerung und/oder zur Visualisierung der Tiefenvorschaukarte (10) in Form von Iso-Tiefenlinien.

7. Mikroskopie-Verfahren, bei welchem mit einem Mikroskop (2) ein mikroskopisches Bildes eines zu untersuchenden Beobachtungsbereiches (1) erzeugt und mit einer Anzeigeeinheit (60) visualisiert wird,
**dadurch gekennzeichnet, dass**
- die dreidimensionale Struktur eines Beobachtungsobjekts (12) von vorhandenen Daten auf die Position des Beobachtungsobjekts in dem Beobachtungsbereich (1) registriert wird,
- aus vorhandenen Daten eine Tiefenvorschaukarte (10) einer dreidimensionalen Struktur des Beobachtungsobjekts (12) berechnet wird, und
- die berechnete dreidimensionale Struktur in Bezug auf die Position des Beobachtungsobjekts (12) in dem Beobachtungsbereich (1) mit Hilfe der Anzeigeeinheit (60) visualisiert wird.

8. Mikroskopie-Verfahren nach Anspruch 7,
**dadurch gekennzeichnet, dass**
die Topographie des Beobachtungsbereiches (1) erfasst wird.

9. Mikroskopie-Verfahren nach Anspruch 7 oder Anspruch 8,
**dadurch gekennzeichnet, dass**
erzeugte mikroskopische Bilder des zu untersuchenden Beobachtungsbereiches (1) erfasst werden und diese mit der Tiefenvorschaukarte (10) kombiniert werden.

10. Mikroskopie-Verfahren nach einem der Ansprüche 7 bis 9,
**dadurch gekennzeichnet, dass**
die Tiefenvorschaukarte (10) als opake Überlagerung und/oder in Form von Iso-Tiefenlinien visualisiert wird.
